# EUROPEAN PATENT SPECIFICATION

(11) **EP 4 548 358 B1**
(45) Date of publication and mention of the grant of the patent: **21.01.2026**
(21) Application number: 22743895.9
(22) Date of filing: 29.06.2022
(51) Int. Cl.: G16H 10/60, G16H 10/65, G16H 40/20

(54) **MEDICAL RECORD SYSTEM**
MEDIZINISCHES AUFZEICHNUNGSSYSTEM
SYSTÈME D'ENREGISTREMENT MÉDICAL

(43) Date of publication of application: 07.05.2025
(73) Proprietor: CADDI DIAGNOSTICS GMBH, 8049 Zürich (CH)
(72) Inventor: VAN GELDER, Max D., 8049 Zürich (CH)
(74) Representative: PPR AG
(86) International application number: PCT/IB2022/056047
(87) International publication number: WO 2024/003597

(56) References cited:
- WO-A1-2021/237345
- US-A1- 2005 027 995
- US-A1- 2020 082 922
- US-A1- 2020 202 996
- US-B2- 7 640 271

## Description

This invention relates to a medical record system comprising a server, a patient access computer device, a first doctor access computer device, a second doctor access computer device, a patient database, a first doctor database, and a second doctor database, and a cloud network over which the access devices and databases are connected to the server, according to the pre-amble of claim 1.

Such medical record systems are designed for accessing and maintaining patient medical records in which such records are remotely accessible by participating patients and participating doctors. The patient medical records may contain data relating to patient (historical) health data, such as laboratory test results, x-ray and MRI scans, and treatment modalities, such as the prescribed identification and doses of pharmaceuticals.

It is well known that a number of adverse patient care problems arise from the mismanagement or unavailability of patient medical records to doctors. As a result, doctors and other medical care personnel spend a disproportionate time to document patient care data, often duplicating data in principle already available. Moreover, in the vast majority of emergency department visits critical (historical) medical data on the patient is not available to the medical personnel on duty. Non-availability of (historical) patient medical data to a doctor may lead to misdiagnosis, to administering contra-indicated drugs, and to redundant laboratory testing, adding to the ever-rising annual medical costs.

In order to improve efficient patient information shared between departments in the health industry, a number of computerized systems for tracking patient medical records have been proposed. As an example, US2003/0088441A1 provides a secure network and server system that allows physicians, patients, hospitals, and insurance companies to input, update and share information, and access it from any location connected to the network. The relevant patient medical records are accessed via a CD-ROM, while the records are being stored at a central server. A disadvantage of this system is that it only allows a patient to access his/her medical records via a special kiosk located at the healthcare provider's premises. As another disadvantage, patient medical data collected by other (off-site) healthcare providers, for instance in other cities or countries, is not available in the system provided. As another example, WO2004/044778A1 discloses an electronic medical record system providing unlimited patient access to one's medical records, wherein the patient caries the medical records on his/her person, for example stored on a portable data memory device, such as a CD-ROM. A disadvantage of this system is that all data is stored on a single portable memory device access to which is not always available. Laboratory results on a biopsy, for instance, usually become available with a delay relative to the time of sampling. The patient carrying his/her medical record may have travelled internationally in the meantime. Thus, upon visiting a foreign doctor abroad, even while carrying the portable data memory with the patient's medical record, the data available to the doctor may not be up-to-date. As yet a further example, US7640271B2 discloses a network-based health care record system providing patient and medical personnel access to all (historical) patient medical records, wherein the access is controlled by the medical personnel. A serious disadvantage of the proposed system is the security and confidentiality of the patient's medical records. Moreover, the system does not take into account that the patient may have different IDs. As is well known, administrative systems differ considerably from country to country, and a single patient may have multiple country-specific IDs with which the patient is identifiable in the respective country-specific health care infrastructures, which include general practitioners, hospitals, medical specialists, and health care insurance companies. Hence, available patient health data accessible to doctors within a first country may not be available to doctors in a second country, simply due to the fact that the patient's first country ID is not usable for identification in the second country.

A disadvantage of the known medical record systems is that they do provide access to a treating doctor of only a limited amount of data of a patient's complete historical and up-to-date medical record. The main reason being that the access to the data in the known systems is driven and controlled by the health care industry, rather than by the patient.

The inventors recognize the need of a medical record system which provides access to a patient's complete historical and up-to-date medical records while safeguarding the security and confidentiality of these records. The invention intends to alleviate at least one of the above-mentioned drawbacks of the prior art, respectively to provide a solution to the identified needs.

The objective of the invention is solved by the features of the independent claims. Advantageous further developments are shown in the figures and in the dependent claims.

According to an aspect of the invention, a medical record system for accessing and maintaining patient medical records, and in which such records are remotely accessible by participating patients and participating doctors is disclosed, the system comprising a server; a first patient access computer device and a first patient database; a first doctor access computer device and a first doctor database for storing first doctor's medical records relating to the first doctor's patients; a second doctor access computer device and a second doctor database for storing second doctor's medical records relating to the second doctor's patients; and a cloud network over which the access devices and databases are connected to the server. The cloud network is arranged to allow communication between the server, the connected devices and the databases. Subject to the first doctor and second doctor being identified as a trusted doctor in a first patient trusted doctor file, the server comprises a two-way firewall arranged to allow the first patient access computer device to obtain and store links for assessing medical records associated with the first patient, and to allow the first doctor access computer device to access the medical records associated with the first patient, irrespective of their stored database location, through the stored links. Advantageously, the patient has discretionary control over who has access to his/her medical records by updating a doctor's status in the patient's trusted doctor file. Moreover, once a doctor has been approved as trusted on a patient's trusted doctor record, the patient does not need to remember which medical health professionals have been visited for consultation / treatment previously. For a link with information to the location of the patient's medical records is added to the patient's trusted doctor file. Advantageously, access to the patient's medical history remains available through these links, even when a doctor's status is changed from "trusted" to "mistrusted". Once the link to the medical record is available in for instance the patient's trusted doctor file, the access of the medical record is independent of the doctor's status as "trusted" or "mistrusted"

In an embodiment, the medical record system preferably is arranged such that entries on the first patient medical records have a time stamp, and the two-way firewall is arranged to allow the first doctors access computer device to display a patient record screen incorporating a historical overview of the patient's medical status based on the accessed patient's medical records. Advantageously, the doctor has a complete historical overview of the patient's medical history. Preferably, in displaying the patient record screen the relevance of the entries is weighted on the basis of a weighting factor associated with the entry on the patient's medical record. Preferably, the weighing factor is time dependent. Preferably the time dependence of the weighting factor is depending on the medical condition entered.

In an embodiment, the two-way firewall is preferably arranged to prohibit the first doctor access device to change second doctor's medical records on second doctor database. Advantageously, this ensures the authenticity of the entries on the patient's medical record.

In a further embodiment of the medical record system patient medical records comprise a patient-record part and a physician-record part, and the two-way firewall is arranged to allow the patient's access device access to the patient-record part and arranged to prohibit access to the physician-record part of the medical records associated with the respective patient. Advantageously, this allows providing the patient with information on the medical status or treatment modalities suitable for laymen interpretation in the patient-record part. At the same time, this allows the physician-record part to contain the full and in-depth details of the patient's medical record.

In yet another embodiment of the medical record system both the patient-record part and physician-record part of a patient's medical record are accessible to a doctor access device, subject to the doctor being identified as trusted in the respective patient's trusted doctor record. Advantageously, this allows a doctor actively treating the patient (and hence having a "trusted" status) to obtain the full medical history of the patient, including the details as communicated to the patient. The medical record system therefore provides a treating doctor a better anamnesis, and hence an improved starting point for the treatment of the patient.

In an embodiment, the two-way firewall is arranged as a distributed firewall. Advantageously, this allows definition of security rules/policies to filter all traffic through medical record system irrespective of its origin.

According to another aspect, the invention provides a method for accessing and maintaining patient medical records on a medical record system, the method comprising:
- Storing a first patient trusted doctor file on a first patient database accessible at a first patient access computer device;
- Storing first doctor's patient medical records relating to the first doctor's patients on a first doctor database accessible by a first doctor access computer device;
- Storing second doctor's patient medical records relating to the second doctor's patients on a second doctor database accessible by a second doctor access computer device;
- Connecting the access computer devices and databases over a cloud network via a two-way firewall
- Allowing the two-way firewall, subject to the first doctor and second doctor being identified as a trusted doctor in the first patient trusted doctor file, to have the first patient access computer device to obtain and store links for assessing the first patient medical records associated with the first patient on the first doctor and second doctor databases; and
- Allowing the two-way firewall, subject to the first doctor and second doctor being identified as a trusted doctor in the first patient trusted doctor file, to have the first doctor access computer device to access the first patient medical records associated with the first patient on the first doctor and second doctor databases through the stored links.

Further advantages, features and details of the invention will be apparent from the following description, in which embodiments of the invention are described with reference to the drawings.

The list of reference signs as well as the technical content of the patent claims and figures are part of the disclosure. The figures are described coherently and comprehensively. Identical reference signs indicate identical components, reference signs with different indices indicate functionally identical or similar components.

The figures show:
- Fig. 1: a first embodiment of the medical record system according to the invention;
- Fig. 2: an embodiment of the patient's trusted doctor files according to the invention;

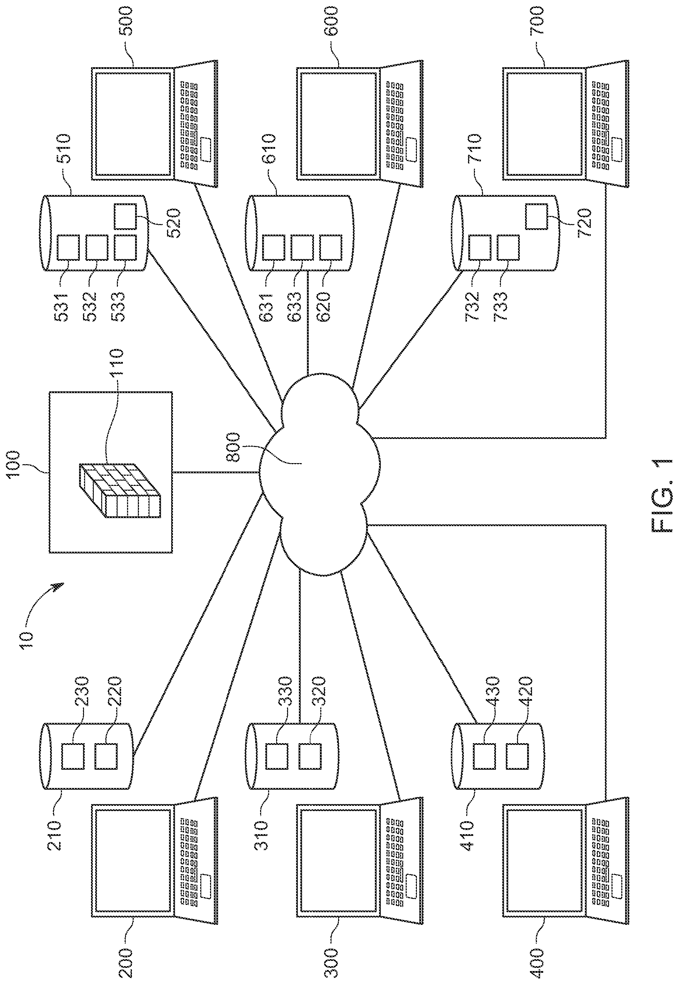

**Fig. 1** schematically shows an example of a medical record system 10 according to the invention. The system comprises a server 100 connectable over a cloud network 800 to a multitude of devices, such as a first patient access device 200 and a first patient memory device or database 210; a second patient access device 300 and a second patient memory device or database 310; a third patient access device 400 and a third patient memory device or database 410; a first doctor (or medical professional) access device 500 and a first doctor memory device or database 510; a second doctor (or medical professional) access device 600 and a second doctor memory device or database 610; and a third doctor (or medical professional) access device 700 and a third doctor memory device or database 710.

A patient may access the medical record system 10 through his/her respective access device 200, 300, 400. These access devices may be computers or mobile devices running appropriate software for connecting to the system through the cloud network, such as the internet. A patient can store information on his/her respective memory device or database 210, 310, 410. These memory devices may be connected to their associated access devices through cloud network 800. Alternatively, they may be integrated in the respective access devices. On each of the memory devices a data record or file 220, 320, 420, is stored comprising personal information identifying the respective patient. The patient's personal information may include name, birthday, email address, sex, social security number(s), insurance number(s), health care provider patient number(s), associated country codes, *etc.* Furthermore, on each patient's memory device a respective trusted doctor file 230, 330, 430 is stored in which the respective patient has recorded, amongst others, the identity of medical professional personnel which are allowed to have access to the patient's medical records. The medical professional personnel may include general practitioners, medical specialists, nurses, pharmacists, insurance personnel, or other appropriately trained personnel. Moreover, the access rights granted to a patient's medical record may be dependent on the role of the medical professional identified in the trusted doctor file. To this end, the patient's trusted doctor file 230, 330, 430 comprises a trusted doctor status indicator associated with medical professional identified.

Medical professional personnel, such as doctors, may access the medical record system 10 through his/her respective access device 500, 600, 700. These access devices may also be computers or mobile devices running appropriate software for connecting to the system through the cloud network, such as the internet. A doctor can store information on his/her respective memory device or database 510, 610, 710. These memory devices may also be connected to their associated access devices through cloud network 800. Alternatively, they may be integrated in the respective access devices. On each of the memory devices a data record or file 520, 620, 720, is stored comprising personal information identifying the respective doctor. The doctor's personal information may include name, birthday, email address, sex, social security number(s), professional identification number, associated health care institution number, professional liability insurance number, *etc.* Furthermore, on each memory device the respective doctors may store patient medical records. For instance, a first doctor, treating (or having treated) patients #1, #2, and #3 may have respective records 531, 532, and 533 associated with these patients. A second doctor, treating patients #1 and #3 may have respective records 631 and 633 associated with these patients. Furthermore, a third doctor, treating patients #2 and #3 may have respective records 732 and 733 associated with these patients.

Server 100 advantageously comprises a two-way firewall 110 program arranged for allowing appropriate access to data records stored in the medial record system 10.

On the one hand, subject to a doctor being identified as a trusted doctor in a patient's trusted doctor file 230, 330, 430, the two-way firewall 110 is arranged to allow the respective patient, using the patient access device 200, 300, 400, to obtain a link to medical records associated with the patient, irrespective where and by whom these medical records have been stored. Thus, as an example and with reference to Fig. 1, via first patient access device 200 patient #1 can access (through a qualified link) first patient medical record 531 on database 510 created by, respectively associated with, doctor #1, and first patient medical record 631 on database 610 created by, respectively associated with, doctor #2, as patient #1 is under consultation / treatment with these doctors and the later are recorded as trusted in first patient trusted doctor file 230. Patient #1 is not allowed to access medical records associated with other patients on these (or other) databases within the network. Similarly, via second patient access device 300 patient #2 can access (through a qualified link) second patient medical record 532 on database 510 created by, respectively associated with, doctor #1, and second patient medical record 732 on database 710 created by, respectively associated with, doctor #3, as the later are recorded as trusted in second patient trusted doctor file 330.

On the other hand, subject to a doctor being identified as a trusted doctor in a patient's trusted doctor file, the two-way firewall 110 is arranged to allow that doctor, using the doctor access device 500, 600, 700, to access medical records associated with that patient, irrespective where and by whom these medical records have been stored. Thus, as an example and with reference to Fig. 1, via first doctor access device 500 doctor #1 can access (through a qualified link) first patient medical record 531 on database 510 created by, respectively associated with, doctor #1 him/herself, as well as first patient medical record 631 on database 610 created by, respectively associated with, doctor #2, as patient #1 is under consultation / treatment with these doctors and the later are recorded as trusted in first patient trusted doctor file 230. Doctor #1 is not allowed to access medical records associated with patients where doctor #1 has not been recorded as a trusted doctor in their respective trusted doctor file, on these (or other) databases within the network. Similarly, via first doctor access device 500 doctor #1 can access (through a qualified link) second patient medical record 532 on database 510 created by, respectively associated with, doctor #1 him/herself, and second patient medical record 732 on database 710 created by, respectively associated with, doctor #3 as patient #2 is under consultation / treatment with these doctors and the later are recorded as trusted in second patient trusted doctor record 330.

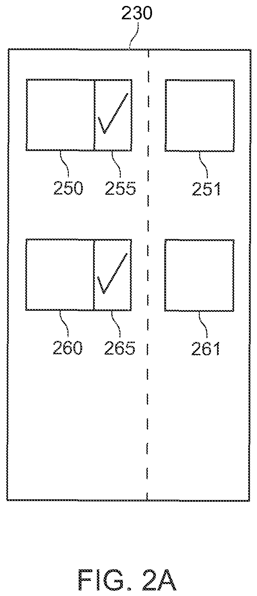

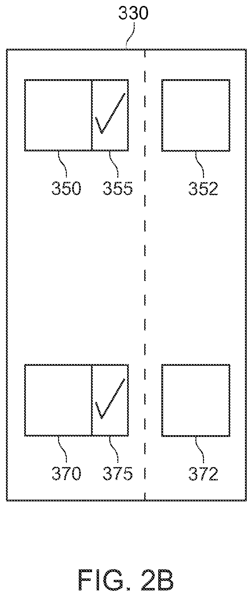

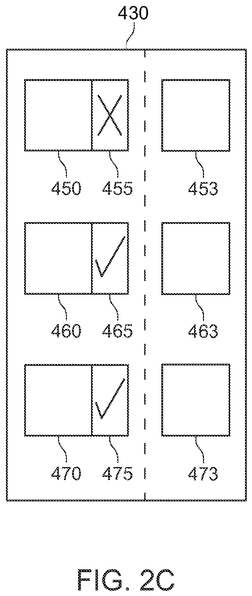

**Fig. 2** schematically shows an embodiment of patient's trusted doctor files 230, 330, 430 associated with respectively patient #1, patient #2, and patient #3. As an example, first patient trusted doctor file 230 comprises as an identification of the first doctor a link 250 to the first doctor ID record 520. These links may have been obtained by the patient from the respective doctors during a preliminary consult. Obtaining the links may for instance be realized through an email exchange. Furthermore, first patient trusted doctor file 230 comprises as an identification of the second doctor a link 260 to the second doctor ID record 620. The status as trusted doctor is provided by the content of first patient first doctor trusted status indicator 255, respectively first patient second doctor trusted status indicator 265. As shown in Fig. 2 on the left-hand side, the first patient's status indicators 255, 265 for the first and second doctor are set to trusted. As a consequence, upon a request from access device 200 to access first patient's medical records, two-way firewall 110 allows first patient to obtain and store a link 251, 261 to his/her medical records 531, 631 created by the first and second doctor, respectively. To this end, the firewall receives from first patient access device 200 the request to obtain a link to the patient's medical records, including an identification of the doctors involved as provided by the content of the links 250, 260 in the first patient trusted doctor file 230, and an identification of these doctors as trusted based on the content of first patient trusted doctor status indicators 255, 265. Upon positive identification, two-way firewall 110 obtains a (hyper-)link defining the first patient's medical records 531, 631 as targets. Subsequently, these links are forwarded and stored in an appropriate location in the first patient's database 210, for instance in the first patient's trusted doctor file 230. Advantageously, once the link to the patient's medical record is available, the medical records remain available to the patient irrespective of the doctor's status as contained in status indicators 255, 265.

Similarly, as a second example shown in the middle of Fig. 2, second patient trusted doctor record 330 comprises links 350, 370 to first doctor ID record 520 and third doctor ID record 720. The links may for instance be obtained through an email exchange between the patient and the respective doctor. As can be discerned from the figure, second patient first doctor trusted status indicator 355 and second patient third doctor trusted status indicator 375 are both set to trusted. As a consequence, upon a request from access device 300 to access second patient's medical records, two-way firewall 110 allows, in a similar fashion as described above, second patient to obtain and store a link 352, 372 to his/her medical records 532, 732 created by the first and third doctor, respectively.

Conversely, as the respective doctors in the first example have been identified as a trusted doctor in the first patient's trusted doctor file 230, the two-way firewall 110 is arranged to allow the first doctor, using the first doctor access device 500, to access (through the links 251, 261) medical records 531, 631 associated with the first patient, irrespective where and by whom these medical records have been stored. Advantageously, the links 251, 261 stored in the first patient's trusted doctor file 230 provides the location information of the respective target medical records. Similarly, the second doctor, using second doctor access device 600, is allowed access to medical records 531, 631 associated with the first patient through the use of links 251 and 261.

Completely analogously, according to the second example, the first doctor is allowed access to medical records 532, 732 associated with the second patient through the use of links 352 and 372. The same example provides the third doctor access to the same medical records 532, 732.

Advantageously, the medical record system according to the invention allows a patient to control access his/her medical records. For this purpose, the content of trusted doctor indicator 255, 265, 355, 375, 455, 465, 475 may be adapted. As a third example, the right-hand side of Fig. 2 shows third patient trusted doctor file 430. As can be taken from the links 450, 460, 470 to the respective doctor ID record 520, 620, 720, patient #3 is or was (not necessarily at the same time) under treatment by the first, second, and third doctor. Moreover, at the time of the respective treatment(s) - and hence the content of the status indicators 455, 465, 475 as trusted - a link 453, 463, 473 to the respective third patient's medical records 533, 633, 733 has been obtained and stored in the third patient trusted doctor file 430. Fig. 2 shows, however, that the third patient's status indicator 455 associated with the first doctor has changed to "mistrusted", while the status indicators 465, 475 associated with the second and third doctor remain "trusted". As a consequence, two-way firewall 110 prohibits the first doctor, using the first doctor access device 500, to access at least medical records 633, 732 associated with the third patient through the links 453, 463, 473. At the same time, the second and third doctors, given their trusted status as provided by indicators 465, 475, remain to have access to the third patient's complete medical history through links 453, 463, 473 - including the (historical) records created by the first doctor.

As will be clear to the person skilled in the art, the embodiments and methods shown in the figures or described herein may also be combined and interchanged within the concept of the invention.

For instance, in a patient's trusted doctor file multiple links may be stored to the patient's medical records created by a single trusted doctor over the course of a treatment or consultation period. The links may advantageously contain a time stamp for reconstructing the medical history at a later date.

As another example, the medial record system is arranged such that a patient's medical history provided to a doctor (recorded as trusted in the patient's trusted doctor file), and updated with the patient's data throughout the system, is provided as a non-storable local copy only available at the time of session accessing the system. Advantageously, this improves the integrity and security of a patient's medical history.

As yet another example, the two-way firewall 100 may be implemented as a distributed firewall. Advantageously, this allows definition of security rules/policies to filter all traffic through medical record system 10, respectively network 800, irrespective of its origin. Server 100 may in this context serve as a centralized management system which pushes out consistent security polies to the end-user devices 200, 300, 400, 500, 600, 700 on the patient and the doctor side of the medical record system 10.

### List of reference signs

- 10: medical record system
- 100: server
- 110: two-way firewall

- 200: first patient access computer device
- 210: first patient database
- 220: first patient personal ID record
- 230: first patient trusted doctor file
- 250: link to first doctor ID record 520
- 251: link to first doctor patient #1 medical record 531
- 255: first patient first doctor trusted status indicator
- 260: link to second doctor ID record 620
- 261: link to second doctor patient #1 medical record 631
- 265: first patient second doctor trusted status indicator

- 300: second patient access computer device
- 310: second patient database
- 320: second patient personal ID record
- 330: second patient trusted doctor file
- 350: link to first doctor ID record 520
- 352: link to first doctor patient #2 medical record 532
- 355: second patient first doctor trusted status indicator
- 370: link to third doctor ID record 720
- 372: link to third doctor patient #2 medical record 732
- 375: second patient third doctor trusted status indicator

- 400: third patient access computer device
- 410: third patient database
- 420: third patient personal ID record
- 430: third patient trusted doctor file
- 450: link to first doctor ID record 520
- 453: link to first doctor patient #3 medical record 533
- 455: third patient first doctor trusted status indicator
- 460: link to second doctor ID record 620
- 463: link to second doctor patient #3 medical record 633
- 465: thirds patient second doctor trusted status indicator
- 470: link to third doctor ID record 720
- 473: link to third doctor patient #3 medical record 733
- 475: third patient third doctor trusted status indicator

- 500: first doctor access computer device
- 510: first doctor database
- 520: first doctor ID record
- 531: first doctor's patient #1 medical record
- 532: first doctor's patient #2 medical record
- 533: first doctor's patient #3 medical record

- 600: second doctor access computer device
- 610: second doctor database
- 620: second doctor ID record
- 631: second doctor's patient #1 medical record
- 633: second doctor's patient #3 medical record

- 700: third doctor access computer device
- 710: third doctor database
- 720: third doctor ID record
- 732: third doctor's patient #2 medical record
- 733: third doctor's patient #3 medical record

- 800: cloud network

## Claims

1. A medical record system (10) for accessing and maintaining patient medical records, and in which such records are remotely accessible by participating patients and participating doctors, the system comprising
a server (100);
a first patient access computer device (200) and a first patient database (210);
a first doctor access computer device (500) and a first doctor database (510) for storing first doctor's patient medical records (531, 532) relating to the first doctor's patients;
a second doctor access computer device (600) and a second doctor database (610) for storing second doctor's patient medical records (631, 633) relating to the second doctor's patients; and
a cloud network (800), over which the access devices (200, 300, 400, 500, 600, 700) and databases (210, 310, 410, 510, 610, 710) are connected to the server, arranged to allow communication between the connected devices (200, 500, 600) and databases (210, 510, 610);
**characterized in that**
the server (100) comprises a two-way firewall (110) arranged, subject to the first doctor and second doctor being identified as a trusted doctor in a first patient trusted doctor file (230), to allow the first patient access computer device (200) to obtain and store links (251, 261) for assessing first patient medical records (531, 631) associated with the first patient, and to allow the first doctor access computer device (500) to access the first patient medical records (531, 631) associated with the first patient through the stored links (251, 261).

2. The medical record system (10) according to claim 1, wherein entries on the first patient medical records (531, 631) have a time stamp, and the two-way firewall (110) is arranged to allow the first doctors access computer device (500) to display a patient record screen incorporating a historical overview of the patient's medical status based on the accessed patient's medical records (531, 631).

3. The medical record system (10) according to claim 1, wherein the two-way firewall (110) is arranged to prohibit the first doctor access device (500) to change second doctor's medical records (631, 633) on second doctor database (610).

4. The medical record system (10) according to claim 1, wherein patient medical records (531, 532, 631, 633, 732, 733) comprise a patient-record part and a physician-record part, and the two-way firewall (110) is arranged to allow the patient's access device (200, 300, 400) access to the patient-record part and arranged to prohibit access to the physician-record part of the medical records associated with the respective patient.

5. The medical record system (10) according to claim 4, wherein both the patient-record part and physician-record part of a patient's medical record (531, 532, 631, 633) are accessible to a doctor access device (500, 600, 700), subject to the doctor being identified as trusted in the respective patient's trusted doctor record (230, 330, 430).

6. The medical record system (10) according to any of the previous claims, wherein the two-way firewall (110) is arranged as a distributed firewall.

7. A method for accessing and maintaining patient medical records on a medical record system (10), the method comprising:
- Storing a first patient trusted doctor file (230) on a first patient database (210) accessible at a first patient access computer device (200);
- Storing first doctor's patient medical records (531, 532) relating to the first doctor's patients on a first doctor database (510) accessible by a first doctor access computer device (500);
- Storing second doctor's patient medical records (631, 633) relating to the second doctor's patients on a second doctor database (610) accessible by a second doctor access computer device (600);
- Connecting the access computer devices (200, 500, 600) and databases (210, 510, 610) over a cloud network (800) via a two-way firewall (110);
- Allowing the two-way firewall, subject to the first doctor and second doctor being identified as a trusted doctor in the first patient trusted doctor file (230), to have the first patient access computer device (200) obtain and store links (251, 261) for assessing the first patient medical records (531, 631) associated with the first patient on the first doctor (510) and second doctor (610) databases; and
- Allowing the two-way firewall, subject to the first doctor and second doctor being identified as a trusted doctor in the first patient trusted doctor file (230), to have the first doctor access computer device (500) access the first patient medical records (531, 631) associated with the first patient on the first doctor (510) and second doctor (610) databases through the stored links (251, 261).

## Patentansprüche

1. Medizinisches Aufzeichnungssystem (10) zum Zugreifen auf und Pflegen von Krankenakten, wobei derartige Akten für teilnehmende Patienten und teilnehmende Ärzte aus der Ferne zugänglich sind, wobei das System Folgendes aufweist:
einen Server (100);
eine Computereinrichtung (200) für den Zugriff eines ersten Patienten und eine erste Patientendatenbank (210);
eine Computereinrichtung (500) für den Zugriff eines ersten Arztes und eine Datenbank (510) des ersten Arztes zum Speichern der Krankenakte (531, 532) des ersten Arztes bezüglich der Patienten des ersten Arztes;
eine Computereinrichtung (600) für den Zugriff eines zweiten Arztes und eine Datenbank (610) des zweiten Arztes zum Speichern der Krankenakte (631, 633) des zweiten Arztes bezüglich der Patienten des zweiten Arztes; und
ein Cloud-Netzwerk (800), über das die Zugriffseinrichtungen (200, 300, 400, 500, 600, 700) und Datenbänke (210, 310, 410, 510, 610, 710) mit dem Server verbunden sind und die derart ausgebildet sind, dass sie eine Kommunikation zwischen den angeschlossenen Geräten (200, 500, 600) und den Datenbänken (210, 510, 610) erlauben;
**dadurch gekennzeichnet, dass**
der Server (100) eine Zwei-Wege-Firewall (110) aufweist, die dazu ausgebildet ist, falls der erste Arzt und der zweite Arzt als Vertrauensärzte in einer Vertrauensarztdatei (230) eines ersten Patienten identifiziert sind, der Computereinrichtung (200) für den Zugriff des ersten Patienten zu erlauben, Links (251, 261) für den Zugriff auf die erste, dem ersten Patienten zugeordnete Krankenakte (531, 631) zu empfangen und zu speichern, und der Computereinrichtung (500) für den Zugriff des ersten Arztes zu erlauben, durch die gespeicherten Links (251, 261) auf die erste, dem ersten Patienten zugeordnete Krankenakte (531, 631) zuzugreifen.

2. Medizinisches Aufzeichnungssystem (10) nach Anspruch 1, wobei die Eingaben in die Krankenakte (531, 631) des ersten Patienten eine Zeitkennung aufweisen, und die Zwei-Wege-Firewall (110) derart ausgebildet ist, dass sie der Computereinrichtung (500) für den Zugriff des ersten Arztes erlaubt, eine Darstellung der Krankenakte, einschließlich einer historischen Übersicht der medizinischen Situation des Patienten, auf Basis der zugegriffenen Krankenakte (531, 631) anzuzeigen.

3. Medizinisches Aufzeichnungssystem (10) nach Anspruch 1, wobei die Zwei-Wege-Firewall (110) dazu ausgebildet ist, die Einrichtung (500) für den Zugriff des ersten Arztes daran zu hindern, die Krankenakte (631, 633) des zweiten Arztes in der Datenbank (610) des zweiten Arztes zu ändern.

4. Medizinisches Aufzeichnungssystem (10) nach Anspruch 1, wobei die Patienten-Krankenakte (531, 532, 631, 633, 732, 733) eine Teilakte Patient und eine Teilakte Arzt enthält, und die Zwei-Wege-Firewall (110) dazu ausgebildet ist, der Einrichtung (200, 300, 400) für den Zugriff des Patienten zu erlauben, auf die Teilakte Patient zuzugreifen, und dazu ausgebildet ist, einen Zugriff auf die Teilakte Arzt der dem jeweiligen Patienten zugeordneten Krankenakte zu verhindern.

5. Medizinisches Aufzeichnungssystem (10) nach Anspruch 4, wobei eine Einrichtung für den Zugriff eines Arztes (500, 600, 700) sowohl auf die Teilakte Patient als auch auf die Teilakte Arzt der Krankenakte (531, 532, 631, 633) zugreifen kann, falls der Arzt in der Vertrauensarztakte (230, 330, 430) des jeweiligen Patienten identifiziert ist.

6. Medizinisches Aufzeichnungssystem (10) nach einem der vorstehenden Ansprüche, wobei die Zwei-Wege-Firewall (110) als verteilte Firewall ausgebildet ist.

7. Verfahren zum Zugreifen auf und Pflegen von Krankenakten in einem medizinischen Aufzeichnungssystem (10), wobei das Verfahren Folgendes umfasst:
- Speichern einer Vertrauensarztdatei (230) für den ersten Patienten in einer ersten Patientendatenbank (210), die an einer Computereinrichtung (200) für den Zugriff eines ersten Patienten zugänglich ist;
- Speichern der Krankenakte (531, 532) eines ersten Arztes bezüglich der Patienten des ersten Arztes in einer Datenbank (510) des ersten Arztes, die für eine Computereinrichtung (500) für den Zugriff des ersten Arztes zugänglich ist;
- Speichern der Krankenakte (631, 633) eines zweiten Arztes bezüglich der Patienten des zweiten Arztes in einer Datenbank (610) des zweiten Arztes, die für eine Computereinrichtung (600) für den Zugriff des zweiten Arztes zugänglich ist;
- Verbinden der Computereinrichtungen (200, 500, 600) für den Zugriff und der Datenbänke (210, 510, 610) über ein Cloud-Netzwerk (800) und eine Zwei-Wege-Firewall (110);
- der Zwei-Wege-Firewall erlauben, falls der erste Arzt und der zweite Arzt als Vertrauensärzte in der Vertrauensarztdatei (230) des ersten Patienten identifiziert sind, dass die Computereinrichtung (200) für den Zugriff des ersten Patienten Links (251, 261) für den Zugriff zu der ersten, dem ersten Patienten zugeordneten Krankenakte (531, 631) in den Datenbänken des ersten Arztes (510) und des zweiten Arztes (610) empfängt und speichert; und
- der Zwei-Wege-Firewall erlauben, falls der erste Arzt und der zweite Arzt als Vertrauensärzte in der Vertrauensarztdatei (230) des ersten Patienten identifiziert sind, dass die Computereinrichtung (500) für den Zugriff des ersten Arztes auf die erste, dem ersten Patienten zugeordnete Krankenakte (531, 631) in den Datenbänken des ersten Arztes (510) und des zweiten Arztes (610) durch die gespeicherten Links (251, 261) zugreift.

## Revendications

1. Système de dossiers médicaux (10) permettant d'accéder à des dossiers médicaux de patients et de les tenir à jour, et dans lequel ces dossiers sont accessibles à distance par des patients participants et des médecins participants, le système comprenant
un serveur (100) ;
un dispositif informatique d'accès de premier patient (200) et une première base de données de premier patient (210) ;
un dispositif informatique d'accès de premier médecin (500) et une base de données de premier médecin (510) pour stocker les dossiers médicaux (531, 532) des patients du premier médecin relatifs aux patients du premier médecin ;
un dispositif informatique d'accès de second médecin (600) et une base de données de second médecin (610) pour stocker les dossiers médicaux du second médecin (631, 633) relatifs aux patients du second médecin ; et
un réseau cloud (800), sur lequel les dispositifs d'accès (200, 300, 400, 500, 600, 700) et bases de données (210, 310, 410, 510, 610, 710) sont connectés au serveur, agencé pour permettre la communication entre les dispositifs connectés (200, 500, 600) et les bases de données (210, 510, 610) ;
**caractérisé en ce que**
le serveur (100) comprend un pare-feu bidirectionnel (110) agencé, sous réserve que le premier médecin et le second médecin soient identifiés comme médecins de confiance dans un premier fichier (230) de médecins de confiance du patient, pour permettre dispositif informatique d'accès du premier patient (200) d'obtenir et de stocker des liens (251, 261) pour évaluer les dossiers médicaux (531, 631) de premier patient associés au premier patient, et l'accès du premier médecin au dispositif informatique (500) aux dossiers médicaux (531, 631) du premier patient associés au premier patient par l'intermédiaire des liens mémorisés (251, 261).

2. Système de dossiers médicaux (10) selon la revendication 1, dans lequel les entrées sur les dossiers médicaux du premier patient (531, 631) ont un horodatage, et le pare-feu bidirectionnel (110) est agencé pour permettre aux premiers médecins d'accéder au dispositif informatique (500) pour afficher un écran de dossier de patient incorporant une vue d'ensemble historique de l'état médical du patient sur la base des dossiers médicaux du patient (531, 631) consultés.

3. Système de dossiers médicaux (10) selon la revendication 1, dans lequel le pare-feu bidirectionnel (110) est agencé pour interdire au dispositif d'accès du premier médecin (500) de modifier les dossiers médicaux du second médecin (631, 633) sur une seconde base de données de médecins (610).

4. Système de dossiers médicaux (10) selon la revendication 1, dans lequel les dossiers médicaux de patient (531, 532, 631, 633, 732, 733) comprennent une partie dossier patient et une partie dossier médecin, et le pare-feu bidirectionnel (110) est agencé pour permettre au dispositif d'accès du patient (200, 300, 400) d'accéder à la partie dossier patient et agencé pour interdire l'accès à la partie dossier médecin des dossiers médicaux associés au patient respectif.

5. Système de dossiers médicaux (10) selon la revendication 4, dans lequel à la fois la partie dossier patient et la partie dossier médecin d'un dossier médical (531, 532, 631, 633) d'un patient sont accessibles à un dispositif d'accès de médecin (500, 600, 700), sous réserve que le médecin soit identifié comme étant de confiance dans le dossier médical de confiance du patient respectif (230, 330, 430).

6. Système de dossiers médicaux (10) selon l'une quelconque des revendications précédentes, dans lequel le pare-feu bidirectionnel (110) est conçu sous forme d'un pare-feu réparti.

7. Procédé permettant d'accéder à des dossiers médicaux de patients et de les conserver sur un système de dossiers médicaux (10), le procédé comprenant les étapes consistant à :
- stocker un fichier de premier médecin de confiance de patient (230) dans une première base de données de patients (210) accessible au niveau d'un dispositif informatique d'accès de premier patient (200) ;
- stocker les dossiers médicaux des patients du premier médecin (531, 532) relatifs aux patients du premier médecin dans une base de données de premier médecin (510) accessible par un dispositif informatique d'accès de premier médecin (500) ;
- stocker les dossiers médicaux des patients du second médecin (631, 633) relatifs aux patients du second médecin dans une base de données de second médecin (610) accessible par un dispositif informatique d'accès de second médecin (600) ;
- connecter les dispositifs informatiques d'accès (200, 500, 600) et les bases de données (210, 510, 610) sur un réseau cloud (800) par l'intermédiaire d'un pare-feu bidirectionnel (110) ;
- permettre au pare-feu bidirectionnel, sous réserve que le premier médecin et le second médecin soient identifiés comme médecins de confiance dans le fichier de premier médecin de confiance (230) du patient, que le premier dispositif informatique d'accès au patient (200) obtienne et stocke des liens (251, 261) pour évaluer les dossiers médicaux du premier patient (531, 631) associés au premier patient dans les bases de données du premier médecin (510) et du second médecin (610) ; et
- permettre au pare-feu bidirectionnel, sous réserve que le premier médecin et le second médecin soient identifiés comme médecins de confiance dans le fichier de premier médecin de confiance (230) du patient, de permettre au premier dispositif informatique d'accès de premier médecin (500) d'accéder aux dossiers médicaux du premier patient (531, 631) associés au premier patient dans les bases de données du premier médecin (510) et du second médecin (610) par l'intermédiaire des liens mémorisés (251, 261).
